(19) Europäisches Patentamt
European Patent Office
Office européen des brevets

(11) EP 3 932 898 A1

(12) **EUROPEAN PATENT APPLICATION**
published in accordance with Art. 153(4) EPC

(43) Date of publication:
**05.01.2022 Bulletin 2022/01**

(21) Application number: **20763864.4**

(22) Date of filing: **21.02.2020**

(51) Int Cl.:
***C07C 41/16*** *(2006.01)* ***C07C 43/23*** *(2006.01)*

(86) International application number:
**PCT/JP2020/007001**

(87) International publication number:
**WO 2020/175353 (03.09.2020 Gazette 2020/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.02.2019 JP 2019034292**
**01.08.2019 JP 2019142209**

(71) Applicant: **Teijin Limited**
**Osaka 530-0005 (JP)**

(72) Inventors:
- **TOMONARI, Yasuhiko**
  **Osaka-shi Osaka 530-0005 (JP)**
- **NUNOME, Kazunori**
  **Osaka-shi Osaka 530-0005 (JP)**

(74) Representative: **Gerauer, Marc Philippé**
**Kraus & Weisert**
**Patentanwälte PartGmbB**
**Thomas-Wimmer-Ring 15**
**DE-80539 München (DE)**

(54) **COMPOUND HAVING BINAPHTHALENE SKELETON AND METHOD FOR PRODUCING COMPOUND HAVING BINAPHTHALENE SKELETON**

(57) A compound having a binaphthalene skeleton represented by the following formula (1):

[Chem. 1]

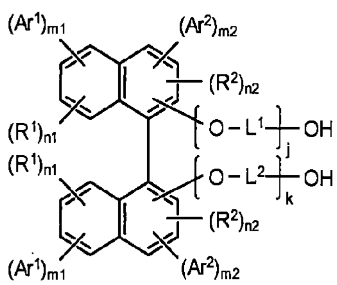

(1)

(wherein $R^1$ and $R^2$ each independently represent a hydrogen atom, a halogen atom or a hydrocarbon group which may contain an aromatic group having 1 to 12 carbon atoms; $Ar^1$ and $Ar^2$ represent an aromatic group which may have a substituent having 6 to 10 carbon atoms; $L^1$ and $L^2$ represent an alkylene group having 1 to 12 carbon atoms; j and k each independently represent an integer of 0 to 5; m1, m2, n1 and n2 each independently represent an integer of 0 to 4, and m1 + m2 ≥ 1, where m1 + n1 is an integer of 4 or less, and m2 + n2 is an integer of 2 or less) , wherein the content of palladium element in the compound having a binaphthalene skeleton satisfies the following formula (2):

$$0 \leq Pd \leq 50 \text{ ppm } (2) \quad (2)$$

This compound having a binaphthalene skeleton has a small content of a specific metal such as palladium or a specific

EP 3 932 898 A1

compound in a raw material alcohol, and is characterized by having excellent hue and various characteristics (optical properties, heat resistance, moldability, etc.) of the raw material and a resin using the raw material.

## Description

### Technical Field

The present invention relates to a compound having a binaphthalene skeleton suitable as a monomer for forming a thermoplastic resin constituting an optical member represented by an optical lens or an optical film, and a method for producing the compound having a binaphthalene skeleton.

### Background Art

In recent years, thermoplastic resin materials such as polycarbonate and polyester made from alcohols having a binaphthalene skeleton represented by 2,2'-bis(2-hydroxyethoxy)-1,1'-binaphthalene (BN2EO) have attracted attention as optical members such as optical lenses and optical sheets because of their excellent optical properties, heat resistance, and moldability. For example, PTL 1 discloses a polyester resin made from an alcohol having a BN2EO as a raw material. Although there is a description that the refraction index of the polyester resin using the alcohol is 1.67, there is a demand for further improvement of the characteristics along with rapid technical innovation in recent years. Thus, with the aim of further increasing the refractive index, PTL 2 has developed a polycarbonate resin in which 9,9-bis[6-(2-hydroxyethoxy)2-naphthyl]fluorene (BNEF) is copolymerized with BN2EO, but there is still room for improvement in the refractive index of the resin described in the patent literature.

Meanwhile, as a method for producing 2, 2'-bis(2-hydroxyethoxy)-1,1'-binaphthalene, a method of reacting 1,1' -bi-2-naphthol with ethylene carbonate described in PTL 3 and a method of reacting 1,1'-bi-2-naphthol with ethylene carbonate or ethylene oxide described in PTL 4 are disclosed, and although a product having high purity and less discoloration can be obtained, optical properties of a thermoplastic resin material using the alcohol as a raw material are not yet satisfactory and there is room for improvement.

In addition, it is described that a compound in which the 6,6'-positions of 2,2'-bis(2-hydroxyethoxy)-1,1'-binaphthalene are substituted is more excellent in optical properties (PTL 5) . PTL 5 discloses a method for synthesizing 2,2'-bis (2-hydroxyethoxy)-6, 6'-diphenyl-1,1'-binaphtha lene. It is described that 2,2'-bis (2-hydroxyethoxy)-6, 6'-diphenyl-1,1'-binaphtha lene obtained by the synthesis method described in the literature has high purity, but there is a problem that the production yield is low because the 2,2'-bis (2-hydroxyethoxy)-6, 6'-diphenyl-1,1'-binaphtha lene is highly purified by repeating purification and washing.

### Citation List

#### Patent Literature

[PTL 1] JP-A-2017-171885
[PTL 2] WO 2018/016516
[PTL 3] Japanese Patent No. 6083900
[PTL 4] Japanese Patent No. 6083901
[PTL 5] WO 2019/043060

### Summary of Invention

#### Technical Problem

An object of the present invention is to provide a compound represented by the following formula (1), which is molecularly designed in the present invention, having a novel binaphthalene skeleton which has a small content of a specific metal such as palladium content and is excellent in hue and various characteristics (optical properties, heat resistance, moldability, etc.) of a raw material thereof and a resin using the raw material.

Another object of the present invention is to provide a method for producing a binaphthalene compound having high purity and excellent hue in high yield.

#### Solution to Problem

The present invention has been achieved as a result of studies for solving the above-mentioned problems of the prior art, and is to provide a compound having a binaphthalene skeleton, which has a certain level of quality and is

excellent as a polymer raw material. Specifically, the present invention relates to a compound having a binaphthalene skeleton shown below.

[1] A compound having a binaphthalene skeleton represented by the following formula (1):

[Chem. 1]

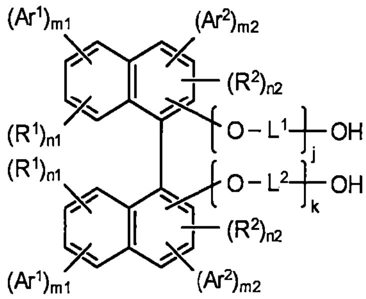

(1)

wherein $R^1$ and $R^2$ each independently represent a hydrogen atom, a halogen atom or a hydrocarbon group which may contain an aromatic group having 1 to 12 carbon atoms; $Ar^1$ and $Ar^2$ represent an aromatic group which may have a substituent having 6 to 10 carbon atoms; $L^1$ and $L^2$ represent an alkylene group having 1 to 12 carbon atoms; j and k each independently represent an integer of 0 to 5; m1, m2, n1 and n2 each independently represent an integer of 0 to 4, and m1 + m2 ≥ 1, where m1 + n1 is an integer of 4 or less, and m2 + n2 is an integer of 2 or less,
wherein the content of palladium element in the compound having a binaphthalene skeleton satisfies the following formula (2) :

$$0 \leq Pd \leq 50 \text{ ppm} \quad (2)$$

[2] The compound having a binaphthalene skeleton according to [1] above, wherein formula (1) is at least one of the following formulae (1a) to (1f):

[Chem. 2]

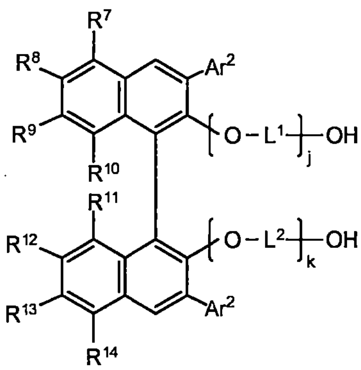

(1a)

[Chem. 3]

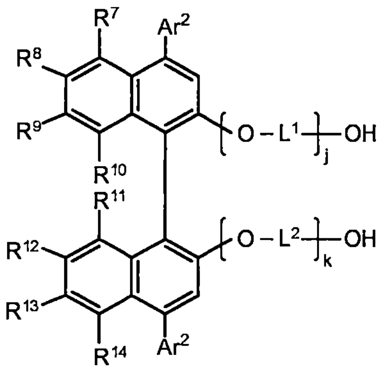

(1 b)

[Chem. 4]

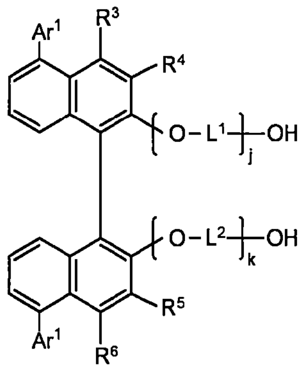

(1 c)

[Chem. 5]

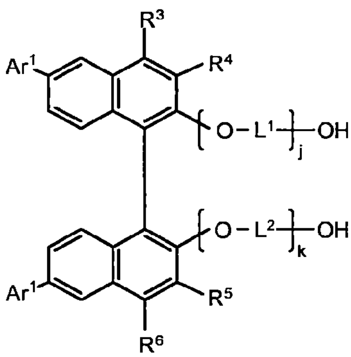

(1 d)

[Chem. 6]

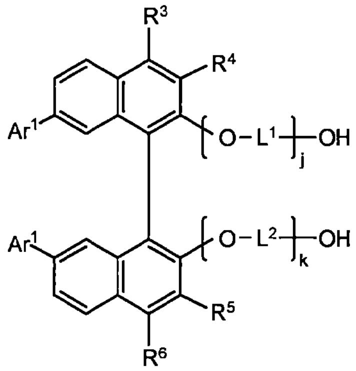

(1e)

[Chem. 7]

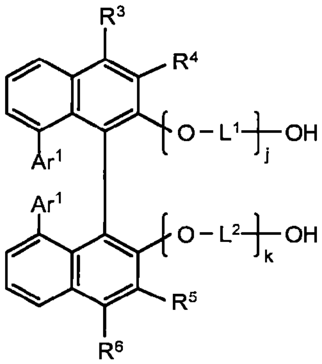

(1f)

wherein $R^3$ to $R^{14}$ each independently represent a hydrogen atom, a halogen atom or a hydrocarbon group which may contain an aromatic group having 1 to 12 carbon atoms, and $Ar^1$ and $Ar^2$, $L^1$ and $L^2$, j and k are the same as those in formula (1).

[3] The compound having a binaphthalene skeleton according to [2] above, wherein formula (1) is formula (1d) .

[4] The compound having a binaphthalene skeleton according to any one of [1] to [3] above, wherein $Ar^1$ or $Ar^2$ in formula (1) is a phenyl group or a naphthyl group.

[5] A method of using the compound having a binaphthalene skeleton according to [1] above as a raw material for a thermoplastic resin.

[6] A method for producing a compound represented by the following formula (II) by reacting a compound represented by the following formula (I) with ethylene carbonate, including the following step 1 and the following step 2:

Step 1: a step of reacting the compound represented by the formula (I) with ethylene carbonate in a using amount (molar ratio) of 1:1.9 to 1:2.9;

Step 2: a step of, after completion of the reaction in step 1, adding an aqueous alkali solution in an amount of 3% by weight or more to the obtained reaction mixture solution, and heating and stirring the mixture at a temperature of 50°C or higher;

[Chem. 8]

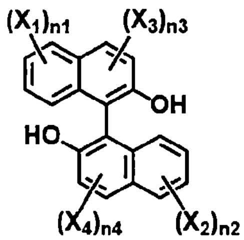

( I )

[Chem. 9]

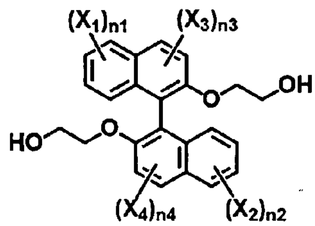

( I I )

wherein $X_1$ to $X_4$ are each independently an aromatic group having 4 to 36 carbon atoms which may contain a hetero atom and may have a substituent, n1 and n2 are each independently an integer of 1 to 4, and n3 and n4 are each independently an integer of 0 to 2.

[7] The method for producing a compound according to [6] above, wherein n1 and n2 in formula (I) and formula (II) are 1, and n3 and n4 in formula (I) and formula (II) are 0.

[8] The method for producing a compound according to [6] or [7] above, wherein formula (II) is the following formula (II-A) :

[Chem. 10]

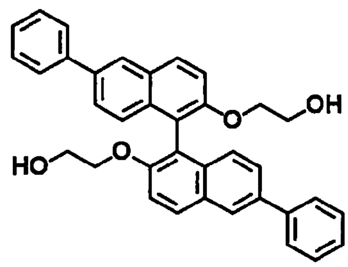

( I I − A)

[9] A compound represented by formula (II-A), wherein a solution obtained by dissolving 0.5 g of a compound represented by the following formula (II-A) in 10 mL of dimethylformamide has an APHA of 100 or less:

[Chem. 11]

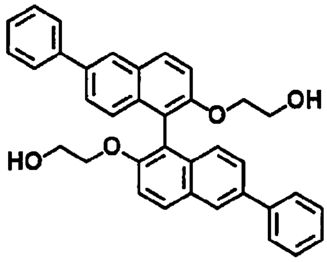

(I I − A)

Advantageous Effects of Invention

Since the binaphthalene compound produced according to the present invention has a high refractive index, and the specific metal content such as palladium content is reduced by optimizing the using amount of a palladium-based catalyst used in the synthesis, the metal can be easily removed by an activated carbon treatment or a metal removal treatment very similar thereto, whereby a compound having a binaphthalene skeleton with a low metal content can be produced with high efficiency. Furthermore, since the binaphthalene compound has two highly reactive hydroxy groups, it can be suitably used as a monomer component of a thermoplastic resin (for example, a polyester resin, a polycarbonate resin, a polyester carbonate resin, or a polyurethane resin).

Further, according to the production method of the present invention, a binaphthalene compound having high purity and excellent hue can be obtained in high yield.

Brief Description of Drawings

Fig. 1 is an NMR spectrum of the compound obtained in Example 1.

Description of Embodiments

<Aspect 1 of the Present Invention>

Although the present invention will be described in detail, the description of the constituent elements described below is a representative example of embodiments of the present invention, and is not limited to the contents thereof.

[Compound Having Binaphthalene Skeleton]

The compound of the present invention is a compound having a binaphthalene skeleton represented by the following formula (1), that is, a compound in which one hydrocarbon having at least one hydroxy group is substituted or added at any of the 2- to 8-positions and 2' - to 8' -positions of the naphthalene rings of the binaphthalene moiety to which the 1,1'-positions of naphthalenes are directly bonded, and further one aromatic group which may have a substituent having 6 to 10 carbon atoms is substituted or added.

[Chem. 12]

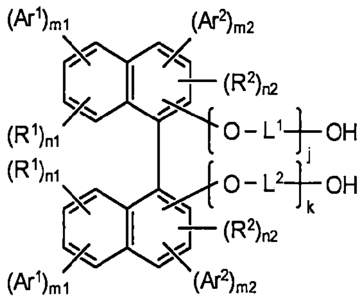

(1)

wherein $R^1$ and $R^2$ each independently represent a hydrogen atom, a halogen atom or a hydrocarbon group which may contain an aromatic group having 1 to 12 carbon atoms; $Ar^1$ and $Ar^2$ represent an aromatic group which may have a substituent having 6 to 10 carbon atoms; $L^1$ and $L^2$ represent an alkylene group having 1 to 12 carbon atoms; j and k each independently represent an integer of 0 to 5; m1, m2, n1 and n2 each independently represent an integer of 0 to 4, and m1 + m2 ≥ 1, where m1 + n1 is an integer of 4 or less, and m2 + n2 is an integer of 2 or less.

In the above formula (1), $R^1$ and $R^2$ each independently represent a hydrogen atom, a halogen atom, or a hydrocarbon group which may contain an aromatic group having 1 to 12 carbon atoms, and are preferably a hydrogen atom, a methyl group, or a phenyl group.

In the above formula (1), examples of the hydrocarbon group represented by $R^1$ and $R^2$ include an alkyl group, a cycloalkyl group, an aryl group, a naphthyl group, and an aralkyl group. Specific examples of the alkyl group preferably include a $C_{1-6}$ alkyl group, a $C_{1-4}$ alkyl group, and a $C_{1-3}$ alkyl group such as a methyl group, an ethyl group, a propyl group, an isopropyl group, a butyl group, and a t-butyl group, and a $C_{1-3}$ alkyl group is more preferable, and a methyl group or an ethyl group is still more preferable.

Specific examples of the cycloalkyl group preferably include a $C_{5-8}$ cycloalkyl group and a $C_{5-6}$ cycloalkyl group such as a cyclopentyl group or a cyclohexyl group, and a $C_{5-6}$ cycloalkyl group is more preferable.

Specific examples of the aryl group preferably include a phenyl group, an alkylphenyl group (such as a mono- or dimethylphenyl group, a tolyl group, a 2-methylphenyl group, and a xylyl group), and a phenyl group is more preferable.

Specific examples of the aralkyl group preferably include a $C_{6-10}$ aryl-$C_{1-4}$ alkyl group such as a benzyl group and a phenethyl group.

The halogen atom is preferably a fluorine atom, a chlorine atom or a bromine atom.

In the above formula (1), the numbers n1 and n2 of the substituents $R^1$ and $R^2$ may be such that n1 is 0 to 4 (for example, 1 to 3), preferably 0 to 2, and more preferably 0 to 1. In addition, n2 may be 0 to 3 (for example, 1 to 3), preferably 0 to 2, and more preferably 0 to 1. n1 and n2 may be the same or different in number in each of the naphthalene rings, or may be the same or different in number in different naphthalene rings. The substituents $R^1$ and $R^2$ are the same as described above, and may be the same or different substituents in the same naphthalene ring and different naphthalene rings, respectively.

In the above formula (1), $L^1$ and $L^2$ each independently represent a divalent linking group, which is an alkylene group having 1 to 12 carbon atoms, more preferably an alkylene group having 1 to 5 carbon atoms, and still more preferably an ethylene group. Usually, $L^1$ and $L^2$ may be the same alkylene group in the binaphthalene ring. Also, $L^1$ and $L^2$ may be the same as or different from each other in different naphthalene rings, and may usually be the same.

The numbers (addition molar numbers) j and k of the oxyalkylene groups ($OL^1$) and ($OL^2$) can each be selected from the range of 0 to 5, and the lower limit is preferably 0 or more, and the upper limit is preferably 4 or less, more preferably 3 or less, and still more preferably 2 or less. It is particularly preferably 0 or 1, and most preferably 1. Note that j and k may be an integer or an average value, and may be the same or different in different naphthalene rings.

In the above formula (1) , $Ar^1$ and $Ar^2$ each independently represent an aromatic group having 6 to 10 carbon atoms, and are preferably a phenyl group or a naphthyl group. $Ar^1$ and $Ar^2$ may be different from each other or the same, but are usually the same. The bonding positions of $Ar^1$ and $Ar^2$ are preferably 3-position and 3'-position, 4-position and 4'-position, 5-position and 5'-position, 6-position and 6'-position, 7-position and 7'-position or 8-position and 8'-position, more preferably 4-position and 4'-position, 6-position and 6'-position or 7-position and 7'-position, and still more preferably 6-position and 6'-position.

In the above formula (1), the substitution numbers m1 and m2 of $Ar^1$ and $Ar^2$ may satisfy m1 + m2 ≥ 1, and for example, m1 may be 0 to 4, preferably 0 to 2, and more preferably 0 to 1. m2 may be 0 to 3, preferably 0 to 2, and more preferably 0 to 1. m1 and m2 may be the same or different in number in each of the naphthalene rings, or may be the same or different in number in different naphthalene rings.

Typical examples of the diol component represented by the above formula (1) are shown below, but the raw materials used in the formula (1) of the present invention are not limited thereto.

Preferred examples of the diphenylbinaphthalene type include

2,2'-bis(2-hydroxyethoxy)-3,3'-diphenyl-1,1'-binaphthalene,
2,2'-bis(2-hydroxyethoxy)-4,4'-diphenyl-1,1'-binaphthalene,
2,2'-bis(2-hydroxyethoxy)-5,5'-diphenyl-1,1'-binaphthalene,
2,2'-bis (2-hydroxyethoxy)-6, 6'-diphenyl-1,1'-binaphtha lene,
2,2'-bis(2-hydroxyethoxy)-7,7'-diphenyl-1,1'-binaphthalene, and
2,2'-bis(2-hydroxyethoxy)-8,8'-diphenyl-1,1'-binaphthalene.

Among them, 2,2'-bis (2-hydroxyethoxy)-6, 6'-diphenyl-1,1'-binaphtha lene represented by the following formula (1-a) is preferable.

Preferred examples of the dinaphthylbinaphthalene type include

2,2'-bis(2-hydroxyethoxy)-3,3'-di-l-naphthyl-1,1'-binaphth alene,
2,2'-bis(2-hydroxyethoxy)-4,4'-di-l-naphthyl-1,1'-binaphth alene,
2,2'-bis(2-hydroxyethoxy)-5,5'-di-1-naphthyl-1,1'-binaphth alene,
2,2'-bis (2-hydroxyethoxy)-6, 6'-di-1-naphthyl-1,1'-bina phth alene,
2,2'-bis(2-hydroxyethoxy)-7,7'-di-1-naphthyl-1,1'-binaphth alene,
2,2'-bis(2-hydroxyethoxy)-8,8'-di-l-naphthyl-1,1'-binaphth alene,
2,2'-bis(2-hydroxyethoxy)-3,3'-di-2-naphthyl-1,1'-binaphth alene,
2,2'-bis(2-hydroxyethoxy)-4,4'-di-2-naphthyl-1,1'-binaphth alene,
2,2'-bis(2-hydroxyethoxy)-5,5'-di-2-naphthyl-1,1'-binaphth alene,
2,2'-bis (2-hydroxyethoxy)-6, 6'-di-2-naphthyl-1,1'-bina phth alene,
2,2'-bis(2-hydroxyethoxy)-7,7'-di-2-naphthyl-1,1'-binaphth alene, and
2,2'-bis(2-hydroxyethoxy)-8,8'-di-2-naphthyl-1,1'-binaphth alene. Among them, 2,2'-bis (2-hydroxyethoxy)-6, 6'-di-2-naphthyl-1,1'-bina phth alene represented by the following formula (1-b) and 2,2'-bis (2-hydroxyethoxy)-6, 6'-di-1-naphthyl-1,1'-bina phth alene represented by the following formula (1-c) are preferable.

[Chem. 13]

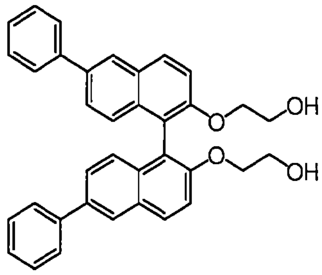

(1 − a)

[Chem. 14]

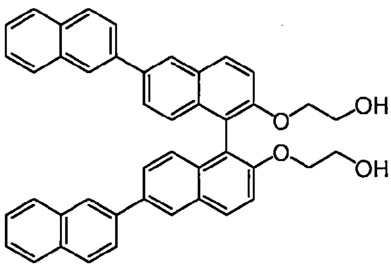

(1 − b)

[Chem. 15]

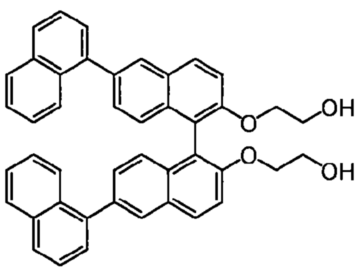

(1 − c)

In the compound having a binaphthalene skeleton of the present invention, the content of palladium element satisfies the following formula (2):

$$0 \leq Pd \leq 50\ ppm\ (2)$$

Preferably, the following formula (2-1) is satisfied:

$$0 \leq Pd \leq 40\ ppm\ (2\text{-}1)$$

More preferably, the following formula (2-2) is satisfied:

$$0 \leq Pd \leq 25\ ppm\ (2\text{-}2)$$

Still more preferably, the following formula (2-3) is satisfied:

$$0 \leq Pd \leq 10\ ppm\ (2\text{-}3)$$

Even more preferably, the following formula (2-4) is satisfied:

$$0 \leq Pd \leq 5\ ppm\ (2\text{-}4)$$

Particularly preferably, the following formula (2-5) is satisfied:

$$0 \leq Pd \leq 3\ ppm\ (2\text{-}5)$$

Most preferably, the following formula (2-6) is satisfied:

$$0 \leq Pd \leq 1\ ppm\ (2\text{-}6)$$

If the content of palladium element exceeds the upper limit of the above range, the hue of the resin using the raw material alcohol represented by the above formula (1) and the optical member using the resin are adversely affected, which is not preferable. The lower limit of the content of palladium element may be 0.01 ppm or more, 0.05 ppm or more, or 0.10 ppm or more.

The purity of the compound having a binaphthalene skeleton of the present invention measured by HPLC is preferably 95% or more, more preferably 98% or more, and still more preferably 99% or more.

[Method for Producing Compound Having Binaphthalene Skeleton]

In the present invention, the compound having a binaphthalene skeleton can be produced, for example, by reacting dibromo-binaphthol with ethylene carbonate and reacting the obtained synthesis product with phenylboronic acid or naphthylboronic acid in a reaction solvent in the presence of a base and a palladium-based catalyst.

The palladium-based catalyst can be removed from the obtained reaction product by an activated carbon treatment or a metal removal treatment very similar thereto.

[Characteristics and Uses of Compound Having Binaphthalene Skeleton]

Since the compound having a binaphthalene skeleton of the present invention preferably has a combination of a binaphthalene skeleton and an aryl group, the compound not only has high refractive index and heat resistance but also can reduce birefringence when formed into a polymer. In order to improve the refractive index, 2,2'-bis-(2-hydroxyethoxy)-1,1'-binaphthalene has been used as a compound having a binaphthalene skeleton. However, although this compound has low birefringence, there is room for improvement in the refractive index. On the other hand, the compound having a binaphthalene skeleton of the present invention has a low birefringence and a high refractive index efficiently, probably because a diaryl group is introduced into the binaphthalene skeleton. Furthermore, since the binaphthalene ring has one or more hydroxyl groups and the binaphthalene compound as a whole has a plurality of hydroxyl groups,

the binaphthalene compound has high reactivity. Therefore, the compound having a binaphthalene skeleton of the present invention can be used as a raw material (monomer) for various resins. For example, it can be used as a polyol component of a thermoplastic resin (for example, a polyester resin, a polycarbonate resin, a polyester carbonate resin, or a polyurethane resin) or a thermosetting resin (for example, an epoxy resin, a phenol resin, a thermosetting polyurethane resin, or a (meth) acrylate ( (meth) acrylic acid ester) ) . When the compound having a binaphthalene skeleton of the present invention is used as a polyol component, the obtained resin has an advantage that both a high refractive index and low birefringence can be achieved at a high level probably because the compound has a diaryl group at the 6,6'-positions of the binaphthalene skeleton.

In addition, derivatives of the compound having a binaphthalene skeleton of the present invention can be efficiently prepared in a general-purpose solvent.

The melting point of the compound having a binaphthalene skeleton of the present invention can be selected from a wide range of 50 to 200°C, and is preferably 70 to 180°C, more preferably 80 to 160°C, and still more preferably 90 to 150°C.

<Aspect 2 of the Present Invention>

<<Process for Producing Binaphthalene Compound>>

(Step 1)

In the present invention, a compound represented by the following formula (I) is reacted with a predetermined amount of ethylene carbonate to obtain a compound represented by the following formula (II):

[Chem. 16]

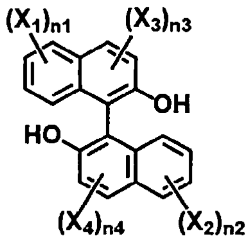

( I )

[Chem. 17]

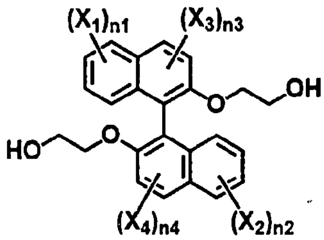

( I I )

wherein $X_1$ to $X_4$ are each independently an aromatic group having 4 to 36 carbon atoms which may contain a hetero atom and may have a substituent, n1 and n2 are each independently an integer of 1 to 4, and n3 and n4 are each independently an integer of 0 to 2.

$X_1$ to $X_4$ in the formula (I) and the formula (II) are each independently an aromatic group having 4 to 36 carbon atoms which may contain a hetero atom and may have a substituent. Examples of the aromatic group having 4 to 36 carbon atoms, preferably 5 to 24 carbon atoms, and more preferably 6 to 18 carbon atoms, and which may contain a hetero atom and may have a substituent include a phenyl group, a naphthyl group, an anthryl group, a phenanthryl group, a phenalenyl group, a fluorenyl group, an acenaphthylenyl group, an acenaphthenyl group, a biphenylenyl group, an indacenyl group, a pyridyl group, a pyrrolidinyl group, and a thienyl group, with a phenyl group and a naphthyl group

being particularly preferred.

Further, n1 and n2 in the formula (I) and the formula (II) are each independently an integer of 1 to 4, preferably an integer of 1 to 2, and particularly preferably 1. Further, n3 and n4 in the formula (I) and the formula (II) are each independently an integer of 0 to 2, preferably an integer of 0 to 1, and particularly preferably 0. That is, the compound represented by the formula (II) is particularly preferably a compound represented by the following formula (II-A).

[Chem. 18]

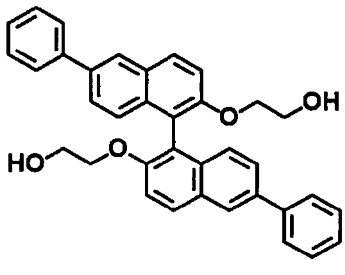

(I I-A)

In the present invention, the using amount (molar ratio) of the compound represented by the formula (I) and ethylene carbonate is 1: 1. 9 to 1:2.9, preferably 1:2 to 1:2.7, and more preferably 1:2.1 to 1:2.5. When the using amount of ethylene carbonate is less than 1:1.9, the reaction time may be prolonged. In addition, the compound represented by the formula (I) remains unreacted, or a by-product obtained by the reaction of 1 mol of the compound represented by the formula (I) with 1 mol of ethylene carbonate increases, whereby the yield and purity decrease, which is not preferable. When the using amount of ethylene carbonate is more than 1:2.9, the amount of a by-product obtained by the reaction of 1 mol of the compound represented by the formula (I) with 3 mols or more of ethylene carbonate becomes large, whereby the yield and purity decrease, which is not preferable.

In the present invention, it is preferable to allow a predetermined amount of a non-reactive solvent to coexist during the reaction. The non-reactive solvent is not particularly limited as long as it does not inhibit the reaction, and examples thereof include aromatic hydrocarbons such as benzene, toluene, xylene, and mesitylene; aliphatic hydrocarbons such as pentane, hexane, and heptane; halogenated aromatic hydrocarbons such as chlorobenzene and dichlorobenzene; halogenated aliphatic hydrocarbons such as dichloromethane and 1,2-dichloroethane; dimethylformamide; and dimethyl sulfoxide. Preferred are toluene and dimethylformamide, and more preferred is dimethylformamide. When a predetermined amount of a non-reactive solvent does not coexist, stirring becomes difficult and the reaction may not proceed or may be significantly delayed. In this case, in order to allow the reaction to proceed, it is necessary to increase the temperature or dilute the reaction system with a large amount of a solvent equal to or greater than a predetermined amount so as to dissolve the reaction system or bring the reaction system into a stirrable slurry state. However, when the reaction temperature is high, a by-product such as a multimer is increased, so that the yield and purity may be lowered or the hue may be deteriorated due to coloring.

In the present invention, the using amount of the non-reactive organic solvent coexisting during the reaction is preferably 0.1 to 10 times by weight, more preferably 0.3 to 7 times by weight, and still more preferably 0.5 to 5 times by weight, relative to the compound represented by the formula (I). When the using amount of the solvent is less than 0.1 times by weight, the compound represented by the formula (I) or the formed compound represented by the formula (II) may be difficult to be stirred. When the using amount of the solvent is more than 10 times by weight, the production efficiency is lowered, for example, the reaction time is delayed or the volume efficiency is lowered, which is economically disadvantageous. In addition, a long-term heating operation may cause an increase in the by-product or coloring. In the present invention, the reaction is preferably carried out using the predetermined amount of ethylene carbonate and the predetermined amount of the non-reactive organic solvent, whereby the compound represented by the formula (I) having a high melting point and low solubility in ethylene carbonate or organic solvent can be most efficiently reacted in a solution or a stirrable slurry state, and the compound represented by the formula (II) can be obtained in high yield and high purity.

In the present invention, the method for reacting the compound represented by the formula (I) with a predetermined amount of ethylene carbonate is not particularly limited, but the reaction can be usually carried out by charging the compound represented by the formula (I) , ethylene carbonate, a solvent and a catalyst into a reaction vessel and heating and stirring them in air or in an inert gas atmosphere such as nitrogen or helium. The reaction can be followed by analytical means such as liquid chromatography.

In the present invention, the reaction temperature is not particularly limited, but is usually 150°C or lower, preferably 140 to 40°C, and more preferably 130 to 70°C. If the reaction temperature is too high, it may cause a decrease

in yield or a deterioration in hue due to an increase in the by-product. If the reaction temperature is too low, the reaction may not proceed rapidly.

The catalyst used in the present invention may be either an alkali catalyst or an acid catalyst, but an alkali catalyst is preferred from the viewpoint of rapid progress of the reaction and reduction of impurities. Examples of the alkali catalyst include potassium hydroxide, sodium hydroxide, barium hydroxide, magnesium oxide, sodium carbonate, and potassium carbonate. Among them, potassium hydroxide, sodium hydroxide, and potassium carbonate are preferable. The case of using an acid catalyst is also not particularly limited, and examples thereof include sulfuric acid, p-toluenesulfonic acid, and methanesulfonic acid. The using amount of the catalyst is not particularly limited, but is usually preferably 0.01 to 0.5 mols, and more preferably 0.05 to 0.2 mols with respect to 1 mol of the compound represented by the formula (I). When the amount of the catalyst is small, the reaction may not proceed or the reaction may be delayed. When the amount of the catalyst is large, the yield and purity may be lowered due to an increase in the by-product, and coloring may be caused.

(Step 2)

In the present invention, a step of adding an aqueous alkali solution having a concentration of 3% by weight or more to a reaction mixture solution containing the compound represented by the formula (II), obtained by reacting the compound represented by the formula (I) with a predetermined amount of ethylene carbonate, and heating and stirring the mixture at a temperature of 50°C or higher (hereinafter referred to as an alkali purification step) is performed.

In the present invention, the concentration of the aqueous alkali solution added to the reaction mixture solution containing the compound represented by the formula (II) is 3% by weight or more, preferably 6% by weight or more, and still more preferably 8% by weight or more. By adding an aqueous alkali solution having a concentration of 3% by weight or more and heating and stirring the mixture at a temperature of 50°C or higher, a by-product obtained by reacting 1 mol of the compound represented by the formula (I) with 3 mols or more of ethylene carbonate is decomposed to form a compound represented by the formula (II) . In addition, since the coloring component can be removed in the aqueous alkali solution, the compound represented by the formula (II) can be obtained with high purity and little coloring. When the concentration of the aqueous alkali solution is lower than 3% by weight, the by-product and the coloring component cannot be efficiently removed, which is not preferable. The alkali concentration is not particularly limited as long as it is 3% by weight or more, but the concentration is preferably 50% by weight or less, more preferably 30% by weight or less, and still more preferably 15% by weight or less, from the viewpoint of alkali solubility and ease of handling.

The temperature at which the aqueous alkali solution is heated and stirred is 50°C or higher, preferably 60°C or higher, and more preferably 80°C or higher, and is preferably a temperature equal to or lower than the boiling point of the solvent used, and more preferably 130°C or lower. When the temperature is lower than 50°C, the by-product cannot be removed or cannot be removed efficiently, which is not preferable. On the other hand, when the temperature is higher than 130°C, impurities increase and the purity is lowered and the hue is deteriorated, which is not preferable. The stirring time is not particularly limited, but is preferably 0.5 to 10 hours, more preferably 1 to 9 hours, and still more preferably 2 to 8 hours.

The alkali used in the aqueous alkali solution of the present invention is not particularly limited, and examples thereof include lithium hydroxide, sodium hydroxide, potassium hydroxide, tetramethylammonium hydroxide, tetraethylammonium hydroxide, calcium hydroxide, barium hydroxide, sodium carbonate, and potassium carbonate. Preferred are sodium hydroxide and potassium hydroxide. The using amount of the alkali is not particularly limited, but is usually preferably 0.1 to 20 mols, more preferably 0.2 to 10 mols, and still more preferably 0.3 to 5 mols with respect to 1 mol of the compound represented by the formula (I), in order to efficiently remove a by-product and a coloring component. When the amount of alkali is less than 0.1 mols, the by-product may not be efficiently removed. In addition, the coloring component may not be efficiently removed, which is not preferable. When the amount of the alkali is more than 20 mols, the purity may be lowered and the hue may be deteriorated, which is not preferable.

In the present invention, in the alkali purification step, an aqueous alkali solution may be added to a reaction mixture solution containing the compound represented by the formula (II), followed by heating and stirring, or the reaction mixture solution may be diluted with an organic solvent, followed by addition of an aqueous alkali solution, followed by heating and stirring. It is usually carried out after dilution with an organic solvent. The organic solvent to be diluted is not particularly limited, and examples thereof include aromatic hydrocarbons such as benzene, toluene, xylene, and mesitylene; aliphatic hydrocarbons such as pentane, hexane, and heptane; halogenated aromatic hydrocarbons such as chlorobenzene and dichlorobenzene; dimethylformamide; and dimethyl sulfoxide. Preferred are toluene and dimethylformamide, and more preferred is dimethylformamide. After the alkali purification operation, the aqueous alkali solution can be separated and removed. Further, after the alkali purification operation, other purification operations such as washing with water, adsorption treatment, and filtration may be added.

In general, the compound represented by the formula (II) is more difficult to crystallize as the amount of the by-product formed by addition of 3 mols or more of ethylene carbonate increases, but in the present invention, since the

amount of the by-product is small, crystallization can be easily performed, and crystals of the compound represented by the formula (II) having good hue and purity can be obtained.

In the present invention, the purity of the compound represented by the formula (II) may be further increased by crystallization and purification. The organic solvent used for crystallization and purification is not particularly limited, and examples thereof include aromatic hydrocarbons such as toluene, xylene, and mesitylene; aliphatic hydrocarbons such as hexane and heptane; halogenated aromatic hydrocarbons such as chlorobenzene and dichlorobenzene; alcohols such as methanol, ethanol, propanol, and butanol; ketones such as acetone, methyl ethyl ketone, and methyl isobutyl ketone; esters such as ethyl acetate and butyl acetate; dimethylformamide; and dimethyl sulfoxide. These organic solvents can be used alone or in combination of two or more thereof. These solvents may be newly added, or the solvent used in the alkali purification step may be used as it is without being newly added. The using amount of the organic solvent is not particularly limited, but is usually about 1 time by weight or more, preferably about 1 to 50 times by weight, and more preferably about 3 to 20 times by weight, with regard to the compound represented by the formula (II), from the viewpoint of economy.

The crystallization and purification can be carried out by a general method and is not particularly limited. However, in general, the target crystal can be obtained by heating the mixture for crystallization to a temperature at which the crystal dissolves, for example, 60°C or higher, preferably 80°C or higher, and then cooling the solution to an appropriate temperature, for example, -10 to 30°C. The precipitated crystals are recovered by filtration or the like, washed if necessary, and dried to be isolated. If necessary, the isolated crystals may be purified. Examples of the purification method include recrystallization (recrystallization) and an impurity removal treatment using an adsorbent such as activated carbon.

<<Binaphthalene Compound>>

In the present invention, the purity of the compound represented by the formula (II) is preferably 95% or more, more preferably 97% or more, and still more preferably 99% or more. With respect to the purity of the present invention, % is an area percentage value excluding a solvent in high performance liquid chromatography (HPLC) measurement.

In the present invention, the solution APHA of the compound represented by the formula (II-A) is preferably 100 or less, more preferably 80 or less, and still more preferably 50 or less. If the solution APHA is higher than 100, when the compound is used as an optical resin raw material, the hue of the resin or an optical member using the resin may be adversely affected.

In the present invention, the solution APHA is measured by measuring the transmission of a measurement sample through a solution obtained by dissolving 0.5 g of the compound represented by the formula (II-A) in 10 mL of dimethylformamide using a colorimeter.

## Examples

Hereinafter, the present invention will be described in detail with reference to Examples, but the present invention is not limited to the following Examples as long as the gist of the present invention is not exceeded.

<Aspect 1 of the Present Invention>

In Examples, various measurements were carried out as follows.

(1) HPLC measurement

The compounds obtained in Examples were measured with the following apparatus under the following conditions to determine the purity.

Equipment in use: manufactured by Waters Corporation
Column: ACQUITY UPLC@BEH C18 2.1 × 150 mm
Eluent (volume): dimethylformamide : ultrapure water (0.1 wt% trifluoroacetic acid) = 70/30.

(2) NMR measurement

The compound obtained in Example 1 was measured with the following apparatus and solvent.

Apparatus: JNM-AL400 (400MHz) manufactured by JEOL Ltd.
Solvent: $CDCl_3$

(3) ICP measurement

The amount of palladium element in the compounds obtained in Examples was measured by the following apparatus.

Equipment in use: Agilent Technologies
Apparatus: Agilent 5100 ICP-OES

(4) Refractive index (nD)

Apparatus: Abbe's refractometer DR-M2 manufactured by ATAGO Co., Ltd.
Method: The compounds obtained in Examples were dissolved in dimethyl sulfoxide, and the refractive indices (wavelengths: 589 nm) at 25°C were measured. The solution refractive index was used to approximate the refractive index as a homopolymer.

[Example 1]

<Step 1>

A flask equipped with a stirrer, a condenser and a thermometer was charged with 5 g (11.3 mmols) of 6,6'-dibromo-1,1'-bi-2-naphthol (hereinafter sometimes abbreviated as BN-6Br), 2.3 g (25.9 mmols) of ethylene carbonate, 0.16 g (1.9 mmols) of potassium carbonate and 15 g of toluene, and the mixture was reacted at 110°C for 5 hours. The progress of the reaction was appropriately confirmed by HPLC, and the reaction was terminated after confirming that the residual amount of BN-6Br was 0.1% by weight or less. To the obtained reaction mixture, 65 g of toluene was added to dilute it, and then 8 g of a 10 wt% sodium hydroxide aqueous solution was added thereto, followed by stirring at 85°C for 1 hour, and the aqueous layer was separated and removed. The organic layer was concentrated, dissolved in ethyl acetate, washed with water, and the aqueous layer was separated and removed. Further, hexane was added thereto, and recrystallization was performed as it was, and as a result, a white solid of the target 2,2'-bis (2-hydroxyethoxy)-6, 6'-dibromo-1,1'-binaphthal ene (hereinafter sometimes abbreviated as BN2EO-6Br) was obtained in 3.7 g (yield: 61%, purity: 98. 8%) . The obtained sample was used as it was in the reaction of Step 2.

<Step 2>

Under a nitrogen atmosphere, a flask equipped with a stirrer, a condenser and a thermometer was charged with 3.5 g (6.6 mmols) of BN2EO-6Br obtained in Step 2, 2.10 g (16.5 mmols) of phenylboronic acid, 0.112 g (0.1 mmols) of tetrakis(triphenylphosphine)palladium, 9 mL of a 2M potassium carbonate aqueous solution, 33 mL of toluene, and 12 mL of ethanol, and the mixture was reacted at 80°C for 2 hours. The progress of the reaction was appropriately confirmed by HPLC, and the reaction was terminated after confirming that the residual amount of BN2EO-6Br was 0.1% by weight or less. The obtained reaction mixture was concentrated, a 1M sodium hydroxide aqueous solution was added thereto, and the mixture was extracted with chloroform. Activated carbon was added to the obtained organic layer, and the mixture was stirred for 1 hour. After filtering the activated carbon, the organic layer was concentrated. After concentration, purification was performed by silica gel column chromatography, and as a result, 2.6 g (yield: 75%, purity: 99.2%) of white crystals of the target 2,2-bis (2-hydroxyethoxy)-6, 6'-diphenyl-1,1'-binaphthal ene (hereinafter sometimes abbreviated as BN2EO-6Ph) was obtained. When the amount of residual metals was measured by ICP, Pd was 7 ppm. In addition, the obtained BN2EO-6Ph was analyzed by 1H NMR, and it was confirmed that it was a target product in which the 6, 6'-positions of the binaphthalene ring were substituted with phenyl groups (Fig. 1) . Further, as a result of measuring the refraction index using the obtained BN2EO-6Ph, it was 1.70 in terms of homopolymer.

[Example 2]

In the same manner as in Example 1 except that phenylboronic acid in Step 2 was changed to 2-naphthaleneboronic acid, 3.1 g (yield: 75%, purity: 99.2%) of a white solid of 2,2-bis (2-hydroxyethoxy)-6, 6'-di-2-naphthyl-1,1'-binap htha lene (hereinafter sometimes abbreviated as BN2EO-6(2Np)) was obtained. When the amount of residual metals was measured by ICP, Pd was 7 ppm. As a result of measuring the refraction index using the obtained BN2EO-6(2Np), it was 1.76 in terms of homopolymer.

[Example 3]

In the same manner as in Example 1 except that phenylboronic acid in Step 2 was changed to 1-naphthaleneboronic acid, 2.9 g (yield: 70%, purity: 99.1%) of a white solid of 2,2-bis (2-hydroxyethoxy)-6, 6'-di-1-naphthyl-1,1'-binap htha lene (hereinafter sometimes abbreviated as BN2EO-6(1Np)) was obtained. When the amount of residual metals was measured by ICP, Pd was 8 ppm. As a result of measuring the refraction index using the obtained BN2EO-6(1Np), it was 1.70 in terms of homopolymer.

[Comparative Example 1]

In the same manner as in Example 1 except that the activated carbon treatment was not performed as the Pd-removing treatment in Step 2, 2.6 g (yield: 75%, purity: 99.2%) of a pale yellow solid of BN2EO-6Ph was obtained. When the amount of residual metals was measured by ICP, Pd was 80 ppm.

[Comparative Example 2]

In the same manner as in Example 2 except that the activated carbon treatment was not performed as the Pd-removing treatment in Step 2, 3.1 g (yield: 75%, purity: 99.2%) of a pale yellow solid of BN2EO-6(2Np) was obtained. When the amount of residual metals was measured by ICP, Pd was 80 ppm.

[Comparative Example 3]

In the same manner as in Example 3 except that the activated carbon treatment was not performed as the Pd-removing treatment in Step 2, 2.9 g (yield: 70%, purity: 99.1%) of BN2EO-6(1Np) was obtained. When the amount of residual metals was measured by ICP, Pd was 80 ppm.

<Aspect 2 of the Present Invention>

In Examples, various measurements were carried out as follows.

(I) High performance liquid chromatography (HPLC) measurement

The measurement was performed using a high performance liquid chromatograph L-2350 manufactured by Hitachi, Ltd., under the measurement conditions shown in Table 1. In the examples, unless otherwise specified, % is an area percentage value corrected by excluding a solvent in HPLC.

[Table 1]

| Column | | Nomura Chemical Develosil ODS-MG-5 | |
|---|---|---|---|
| Column temperature | °C | 40 | |
| Detection wavelength | nm | 253 | |
| Flow rate | mL/min | 1 | |
| Injection amount | μL | 10 | |
| Eluent A | - | acetonitrile | |
| Eluent B | - | 0.1% TFA aq. | |
| Gradient | | Yes | |
| Time | min | A (%) | B (%) |
| 0 | | 30 | 70 |
| 5 | | 30 | 70 |
| 60 | | 85 | 15 |
| 65 | | 100 | 0 |

(continued)

| Column | Nomura Chemical Develosil ODS-MG-5 | |
|---|---|---|
| 80 | 100 | 0 |
| 81 | 30 | 70 |

(II) APHA measurement

A solution obtained by dissolving 0 . 5g of the measurement sample in 10 mL of dimethylformamide was put into a φ25 mm test tube, and measurement was performed using TZ6000 manufactured by Nippon Denshoku Industries Co., Ltd.

[Example 4]

(Step 1)

A flask equipped with a stirrer, a condenser and a thermometer was charged with 40.00 g (0.091 mols) of 6,6'-diphenyl-1,1'-bi-2-naphthol (hereinafter sometimes abbreviated as BN-6Ph), 18.47 g (0.210 mols) of ethylene carbonate, 1.31 g of potassium carbonate and 40 mL of dimethylformamide, and the mixture was reacted at 120°C for 9 hours. As a result of measurement by HPLC, BN-6Ph was 0.1%, 2,2'-bis (2-hydroxyethoxy)-6, 6'-diphenyl-1,1'-binaphtha lene (hereinafter sometimes abbreviated as BN2EO-6Ph) was 95.1%, and the by-product obtained by reacting 3 mols or more of ethylene carbonate with respect to 1 mol of BN-6Ph was 3.0%.

(Step 2)

To the reaction mixture solution obtained in Step 1, 60 mL of dimethylformamide was added to dilute it, and then 9 mL of a 10 wt% sodium hydroxide aqueous solution was added thereto, followed by stirring at 110°C for 4 hours, and then the reaction solution was added dropwise into 2L of distilled water while stirring to crystallize BN2EO-6Ph. The crystals were recovered and subjected to slurry washing three times with 2L of distilled water. The recovered crystals were vacuum-dried at 90°C for 7 hours to obtain 46 g of BN2EO-6Ph crystals (yield: 96%, purity: 99.1%, by-product obtained by reaction of 3 mols or more of ethylene carbonate with respect to 1 mol of BN-6Ph: 0.0%, APHA: 50).

[Example 5]

(Step 1)

A flask equipped with a stirrer, a condenser and a thermometer was charged with 35.00 g (0.080 mols) of BN-6Ph, 16.16 g (0.184 mols) of ethylene carbonate, 1.15 g of potassium carbonate and 35 mL of toluene, and the mixture was reacted at 110°C for 11 hours. As a result of measurement by HPLC, BN-6Ph was 0.1%, BN2EO-6Ph was 91.5%, and the by-product obtained by reacting 3 mols or more of ethylene carbonate with respect to 1 mol of BN-6Ph was 4.2%.

(Step 2)

To the obtained reaction mixture solution, 50 mL of toluene was added to dilute it, and then 8 mL of a 10 wt% sodium hydroxide aqueous solution was added thereto, followed by stirring at 110°C for 4 hours. After completion of the reaction, the reaction solution was transferred to a separatory funnel and washed with distilled water until the reaction solution became neutral. After the reaction solution was concentrated, recrystallization was carried out once with methyl ethyl ketone to obtain 39 g of BN2EO-6Ph crystals (yield: 93%, purity: 98.6%, by-product obtained by reaction of 3 mols or more of ethylene carbonate with respect to 1 mol of BN-6Ph: 0.0%, APHA: 30).

[Comparative Example 4]

As a result of HPLC measurement after the reaction in the method described in Example 1.5 of PTL 1 (the condition is that the compound represented by formula (1) and ethylene carbonate are reacted in a using amount (molar ratio) of 1:3, and since a step of adding 3% by weight or more of an aqueous alkali solution and heating and stirring at

a temperature of 50°C or higher is not carried out, claim 1 of the present invention is not satisfied), BN-6Br was 0.1%, BN2EO-6Ph was 92.8%, and the by-product obtained by reacting 3 mols or more of ethylene carbonate with respect to 1 mol of BN-6Ph was 5.3%. Thereafter, washing, purification and drying were carried out by the methods described in Example 1.5 of PTL 1 to obtain BN2EO-6Ph (yield: 69%, purity: 99%, by-product obtained by reacting 3 mols or more of ethylene carbonate with respect to 1 mol of BN-6Ph: 0.5%, APHA: 150) . Since a step of adding 3% by weight or more of an aqueous alkali solution and heating and stirring at a temperature of 50°C or higher was not carried out, and a by-product was removed by washing or recrystallization, the yield was low.

Industrial Applicability

The resin containing the compound having a binaphthalene skeleton of the present invention as a raw material (monomer) can be used for optical members such as films, lenses, prisms, optical disks, transparent conductive substrates, optical cards, sheets, optical fibers, optical films, optical filters, and hard coat films, and is extremely useful particularly for lens applications such as mobile phones.

## Claims

1. A compound having a binaphthalene skeleton represented by the following formula (1):

[Chem. 1]

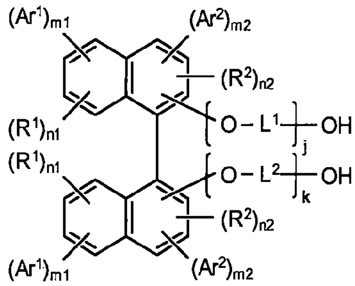

(1)

wherein $R^1$ and $R^2$ each independently represent a hydrogen atom, a halogen atom or a hydrocarbon group which may contain an aromatic group having 1 to 12 carbon atoms; $Ar^1$ and $Ar^2$ represent an aromatic group which may have a substituent having 6 to 10 carbon atoms; $L^1$ and $L^2$ represent an alkylene group having 1 to 12 carbon atoms; j and k each independently represent an integer of 0 to 5; m1, m2, n1 and n2 each independently represent an integer of 0 to 4, and $m1 + m2 \geq 1$, where $m1 + n1$ is an integer of 4 or less, and $m2 + n2$ is an integer of 2 or less,
wherein the content of palladium element in the compound having a binaphthalene skeleton satisfies the following formula (2) :

$$0 \leq Pd \leq 50 \text{ ppm} \quad (2)$$

2. The compound having a binaphthalene skeleton according to claim 1, wherein formula (1) is at least one of the following formulae (1a) to (1f):

[Chem. 2]

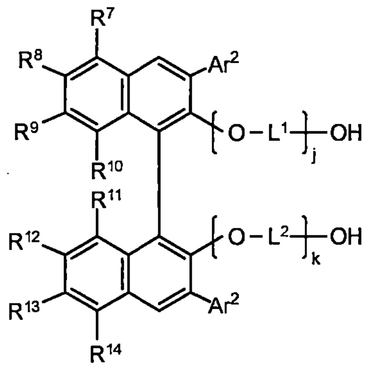

(1 a)

[Chem. 3]

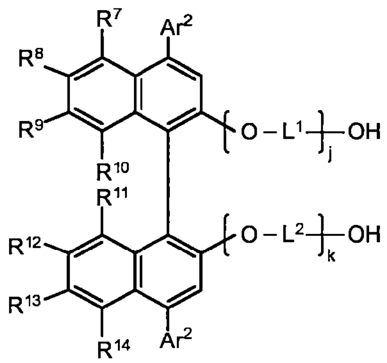

(1 b)

[Chem. 4]

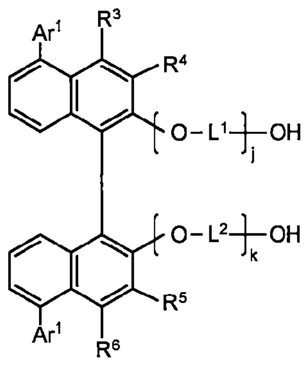

(1 c)

[Chem. 5]

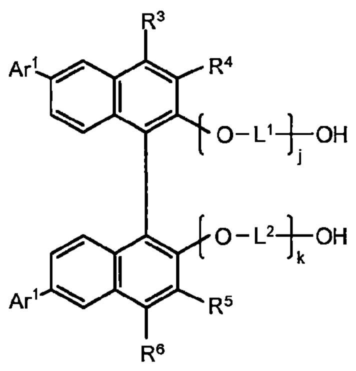

(1 d)

[Chem. 6]

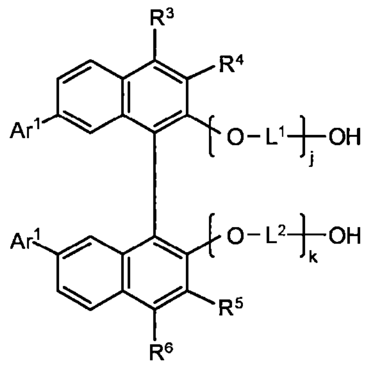

(1 e)

[Chem. 7]

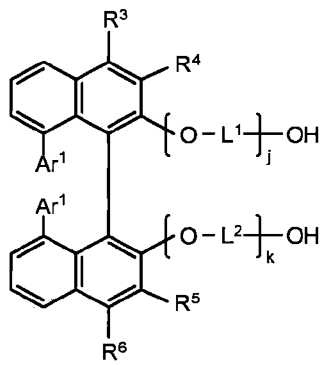

(1 f)

wherein $R^3$ to $R^{14}$ each independently represent a hydrogen atom, a halogen atom or a hydrocarbon group which may contain an aromatic group having 1 to 12 carbon atoms, and $Ar^1$ and $Ar^2$, $L^1$ and $L^2$, j and k are the same as those in formula (1).

**3.** The compound having a binaphthalene skeleton according to claim 2, wherein formula (1) is formula (1d).

**4.** The compound having a binaphthalene skeleton according to any one of claims 1 to 3, wherein $Ar^1$ or $Ar^2$ in formula (1) is a phenyl group or a naphthyl group.

**5.** A method of using the compound having a binaphthalene skeleton according to claim 1 as a raw material for a thermoplastic resin.

**6.** A method for producing a compound represented by the following formula (II) by reacting a compound represented by the following formula (I) with ethylene carbonate, comprising the following step 1 and the following step 2:

Step 1: a step of reacting the compound represented by the formula (I) with ethylene carbonate in a using amount (molar ratio) of 1:1.9 to 1:2.9;
Step 2: a step of, after completion of the reaction in step 1, adding an aqueous alkali solution in an amount of 3% by weight or more to the obtained reaction mixture solution, and heating and stirring the mixture at a temperature of 50°C or higher;

[Chem. 8]

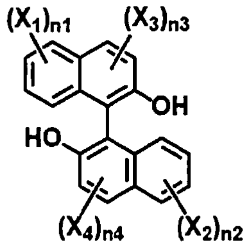

( I )

[Chem. 9]

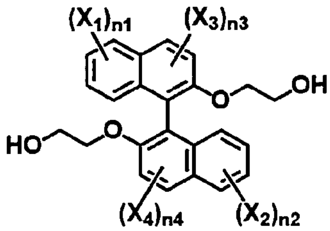

( I I )

wherein $X_1$ to $X_4$ are each independently an aromatic group having 4 to 36 carbon atoms which may contain a hetero atom and may have a substituent, n1 and n2 are each independently an integer of 1 to 4, and n3 and n4 are each independently an integer of 0 to 2.

**7.** The method for producing a compound according to claim 6, wherein n1 and n2 in formula (I) and formula (II) are 1, and n3 and n4 in formula (I) and formula (II) are 0.

**8.** The method for producing a compound according to claim 6 or 7, wherein formula (II) is the following formula (II-A) :

[Chem. 10]

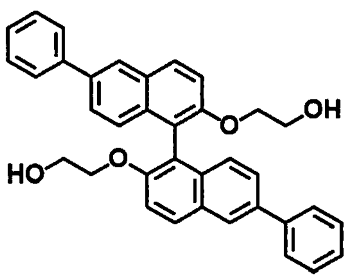

( I I − A )

**9.** A compound represented by formula (II-A), wherein a solution obtained by dissolving 0 . 5 g of a compound represented by the following formula (II-A) in 10 mL of dimethylformamide has an APHA of 100 or less:

[Chem. 11]

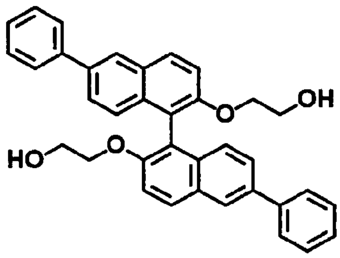

(I I − A)

[FIG. 1]

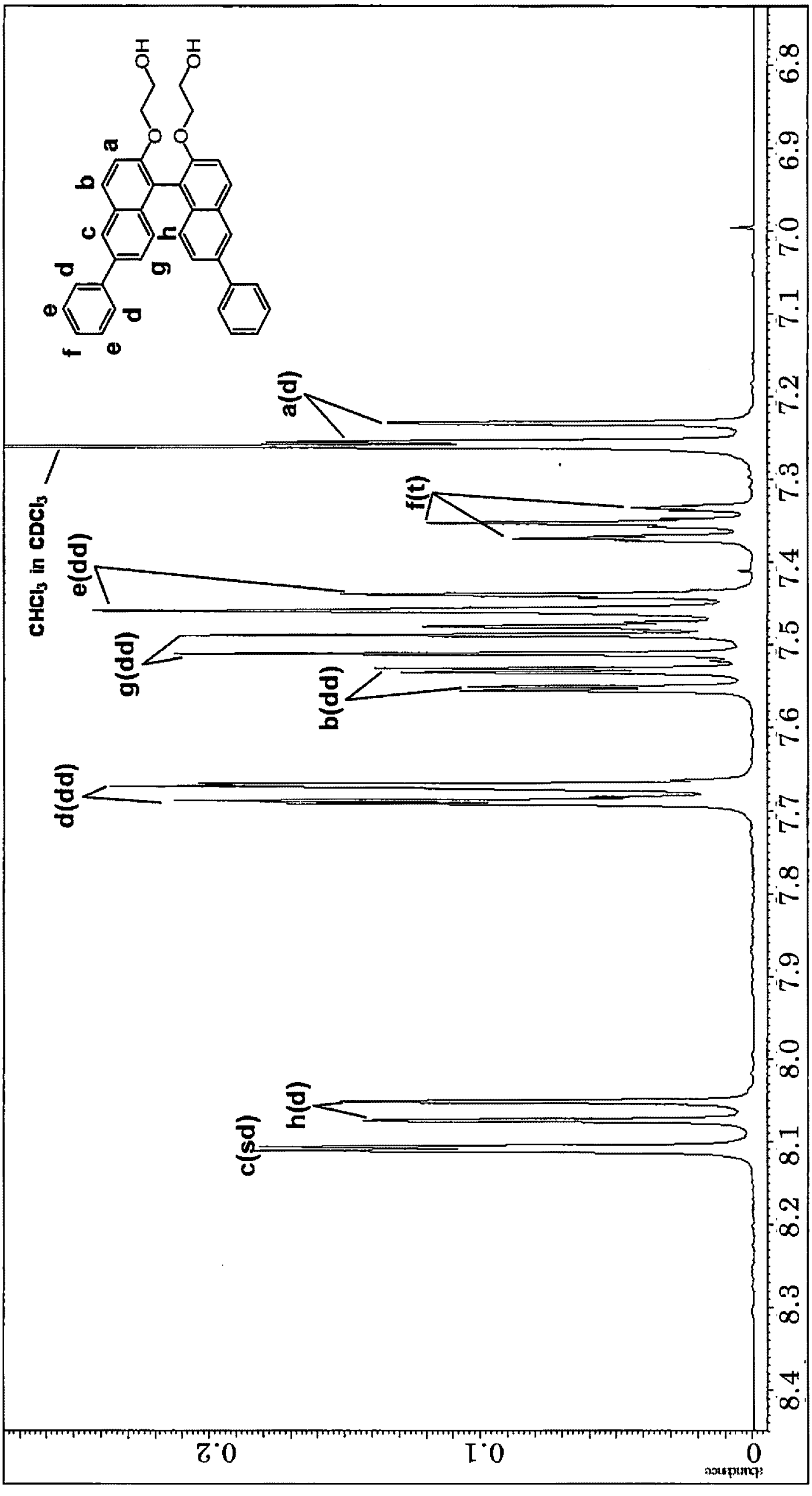
a(d)
CHCl$_3$ in CDCl$_3$
f(t)
e(dd)
g(dd)
b(dd)
d(dd)
h(d)
c(sd)
6.8
6.9
7.0
7.1
7.2
7.3
7.4
7.5
7.6
7.7
7.8
7.9
8.0
8.1
8.2
8.3
8.4
0
0.1
0.2
abundance

## INTERNATIONAL SEARCH REPORT

International application No. PCT/JP2020/007001

A. CLASSIFICATION OF SUBJECT MATTER
C07C 41/16(2006.01)i; C07C 43/23(2006.01)i
FI: C07C43/23 D CSP; C07C43/23 A; C07C41/16
According to International Patent Classification (IPC) or to both national classification and IPC

B. FIELDS SEARCHED

Minimum documentation searched (classification system followed by classification symbols)
C07C41/16; C07C43/23

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY (STN); CASREACT (STN)

C. DOCUMENTS CONSIDERED TO BE RELEVANT

| Category* | Citation of document, with indication, where appropriate, of the relevant passages | Relevant to claim No. |
|---|---|---|
| Y | CN 101992076 A (EAST CHINA UNIVERSITY OF SCIENCE AND TECHNOLOGY) 30.03.2011 (2011-03-30) claims, paragraphs [0017]-[0021] | 1-5 |
| Y | YAN, Yuanyong et al., "Chiral binaphthyl ligands with buttressing substituents", Monatshefte fuer Chemie, 1999, 130(7), 873-885, scheme 1 | 1-2, 4-5 |
| Y | YAN, Yuanyong et al., "Synthesis and application of macrocyclic binaphthyl ligands with extended chiral bias", Tetrahedron: Asymmetry, 1998, 9(20), 3607-3610, scheme 1 | 1-2, 4-5 |
| Y | JP 2015-205235 A (TOSOH CORP.) 19.11.2015 (2015-11-19) claims, examples, etc. | 1-5 |
| Y | JP 50-49189 A (MITSUBISHI CHEM IND LTD.) 01.05.1975 (1975-05-01) claims, examples, etc. | 1-5 |
| Y | JP 50-49188 A (MITSUBISHI CHEM IND LTD.) 01.05.1975 (1975-05-01) claims, examples, etc. | 1-5 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 May 2020 (11.05.2020) | 26 May 2020 (26.05.2020) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No. PCT/JP2020/007001

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-19436 A (BAYER AG.) 21.01.2003 (2003-01-21) claims, paragraph [0074], etc. | 1-5 |
| X | JP 2014-227388 A (TAOKA CHEMICAL CO., LTD.) 08.12.2014 (2014-12-08) claims, examples, paragraphs [0027], [0029] | 6-9 |
| Y | claims, examples, paragraphs [0027], [0029] | 6-9 |
| Y | WO 2019/043060 A1 (REUTER CHEMISCHE APPARATEBAU KG) 07.03.2019 (2019-03-07) claims, examples, etc. | 6-9 |
| P, X | claims, examples, etc. | 1-5 |

Form PCT/ISA/210 (continuation of second sheet) (January 2015)

**INTERNATIONAL SEARCH REPORT**
Information on patent family members

International application No.
PCT/JP2020/007001

| Patent Documents referred in the Report | Publication Date | Patent Family | Publication Date |
|---|---|---|---|
| CN 101992076 A | 30 Mar. 2011 | (Family: none) | |
| JP 2015-205235 A | 19 Nov. 2015 | (Family: none) | |
| JP 50-49189 A | 01 May 1975 | (Family: none) | |
| JP 50-49188 A | 01 May 1975 | (Family: none) | |
| JP 2003-19436 A | 21 Jan. 2003 | US 2002/0173421 A1 claims, paragraph [0128] EP 1260270 A1 | |
| JP 2014-227388 A | 07 Dec. 2014 | (Family: none) | |
| WO 2019/043060 A1 | 07 Mar. 2019 | TW 201914987 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017171885 A
- WO 2018016516 A
- JP 6083900 B
- JP 6083901 B
- WO 2019043060 A